(19) Europäisches Patentamt / European Patent Office / Office européen des brevets

(11) **EP 4 018 229 B1**

(12) **EUROPÄISCHE PATENTSCHRIFT**

(45) Veröffentlichungstag und Bekanntmachung des Hinweises auf die Patenterteilung:
**01.10.2025  Patentblatt 2025/40**

(21) Anmeldenummer: **20734187.6**

(22) Anmeldetag: **23.06.2020**

(51) Internationale Patentklassifikation (IPC):
**G02B 5/00** *(2006.01)*     **G02B 5/04** *(2006.01)*
**G02B 23/24** *(2006.01)*     **C03B 33/02** *(2006.01)*
**A61B 1/00** *(2006.01)*

(52) Gemeinsame Patentklassifikation (CPC):
**G02B 5/005; A61B 1/00096; A61B 1/00179; A61B 1/00188; G02B 5/04; G02B 23/2423**

(86) Internationale Anmeldenummer:
**PCT/EP2020/067428**

(87) Internationale Veröffentlichungsnummer:
**WO 2021/032340 (25.02.2021 Gazette 2021/08)**

(54) **SUBBAUGRUPPE, OBJEKTIV UND LANGES UND SCHLANKES OPTISCHES BILDÜBERTRAGUNGSSYSTEM**

SUB-ASSEMBLY, LENS AND LONG AND THING OPTICAL IMAGE TRANSMISSION SYSTEM

SOUS-MODULE, LENTILLE ET SYSTÈME DE TRANSMISSION DES IMAGES OPTIQUES LONG ET ÉTROIT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2019  EP 19192423**

(43) Veröffentlichungstag der Anmeldung:
**29.06.2022  Patentblatt 2022/26**

(73) Patentinhaber: **Fisba AG**
**9016 St. Gallen (CH)**

(72) Erfinder:
• **SELM, Romedi**
**9300 Wittenbach (CH)**
• **FORRER, Martin**
**9050 Appenzell (CH)**

(74) Vertreter: **Hepp Wenger Ryffel AG**
**Friedtalweg 5**
**9500 Wil (CH)**

(56) Entgegenhaltungen:
CN-A- 105 403 992      DE-A1- 3 640 186
DE-C2- 3 640 186       US-A- 4 815 833
US-A1- 2013 044 361    US-B2- 10 292 574

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Subbaugruppe für ein Objektiv, insbesondere für ein langes und schlankes optisches Bildübertragungssystem, zum Beispiel ein Endoskop, ein Objektiv und ein optisches Bildübertragungssystem, sowie Verfahren zum Herstellen einer Subbaubaugruppe und eines Objektivs.

Lange und schlanke optische Bildübertragungssystems, zum Beispiel Endoskope, sind weit verbreitet, um intrakavitären Organe, intraorganische Wandoberflächen oder andere Ziele durch Einführen eines röhrenförmigen Einsatzes in eine Körperhöhle zu untersuchen und zu diagnostizieren. Auch im industriellen Bereich werden Endoskope eingesetzt, um das Innere von Rohren, Kesseln, Maschinen, Chemieanlagen usw. zu inspizieren.

Derartige Bildübertragungssysteme umfassen in der Regel ein optisches Beleuchtungssystem zum Ausstrahlen von Beleuchtungslicht an der distalen Seite des Bildübertragungssystems und ein Okular auf der proximalen Seite des Bildübertragungssystems, also auf der Seite des Betrachters.

Ein derartiges Bildübertragungssystem kann ein flexibles oder ein starres röhrenförmiges Gehäuse aufweisen.

Einfache Bildübertragungssysteme werden mit einem geraden Blickfeld realisiert. Das heisst, die Achse des Blickfeldes verläuft in Richtung der mechanischen Achse des röhrenförmigen Gehäuses und das Blickfeld ist durch die Form des Gehäuses und die Optik im Gehäuse begrenzt. Viele Anwendungen benötigen jedoch ein vergrössertes Blickfeld.

Bevorzugt weist in diesem Fall das Bildübertragungssystem am distalen Ende ein abgewinkeltes Objektiv auf, sodass die optische Achse der distalen Öffnung einen Winkel mit der Längsachse des Bildübertragungssystems einschliesst.

Für ein Bildübertragungssystem mit flexiblem, röhrenförmigem Gehäuse wird unter der Längsachse die Richtung des gestreckten Gehäuses verstanden.

Zur Ablenkung eines Lichtstrahls im abgewinkelten Objektiv ist zumeist ein Prisma vorgesehen.

Aus US 4,138,192 ist ein Objektiv mit einem Verbundprisma bekannt, bei dem das eigentliche Umlenkprisma von zwei beidseitig angeordneten Prismen gestützt wird, wobei die Prismen aneinander gekittet sind. Solche Objektive sind aufwendig und daher teuer in der Herstellung.

US 4,850,342 zeigt Objektive mit einzelnen Prismen. In diesen Fällen ist aufwendig, eine mechanische Verbindung zu erstellen.

Aus US 5,519,532 ist ein Objektiv mit Einzelprisma bekannt, das aus Kunststoff gefertigt ist. Eine ausreichende optische Abbildungsgüte mit präzisen Reflexionsflächen ist nur schwer zu erreichen.

CN105403992A offenbart eine Objektivlinse für ein Hartröhrenendoskop, die ein Schutzfenster, eine plankonkave Linse, drei verkittete Prismen, eine plankonvexe Linse, eine einzelne Linse, doppelte verkittete Linsen und eine parallele Platte umfasst, welche miteinander verklebt sind.

DE3640186A1 zeigt ein Objektiv für Endoskope mit einer seitlichen Objektivlinse, das aus einem langgestreckten Prisma mit einer konvexen proximalen Endfläche und einem distalen Ende besteht, die nach Ausrichtung auf eine optische Achse durch Verkittung miteinander verbunden sind, und mit einer Blende sowie einer Negativlinse eine unlösbare Einheit bilden.

Es ist die Aufgabe der Erfindung, eine Subbaugruppe, ein Objektiv, ein optisches Bildübertragungssystem, sowie ein Verfahren zum Herstellen einer Subbaubaugruppe und eines Objektivs vorzustellen, welche die Nachteile des Bekannten vermeiden und insbesondere eine leichte Montage und gute Handhabbarkeit bei präzisen Darstellungseigenschaften erlauben.

Die Aufgabe wird gelöst durch die Merkmale der unabhängigen Ansprüche.

Eine Subbaugruppe für ein Objektiv, insbesondere für ein langes und schlankes optisches Bildübertragungssystem, wie zum Beispiel ein Endoskop, umfasst ein Prisma und eine Blendenelement. Das Prisma und das Blendenelement sind fest miteinander verbunden. Bevorzugt ist das Prisma direkt mit dem Blendenelement verbunden, wobei allenfalls Verbindungsmittel, zum Beispiel ein optischer Kitt, direkt zwischen dem Prisma und dem Blendenelement vorgesehen sind.

Bevorzugt ist das Prisma über eine Verkittung oder über ein direktes Bonding, wie hydrophiles Bonden oder laserunterstütztes Bonden, mit dem Blendenelement verbunden.

Beim Kitten werden Komponenten mit polierten Oberflächen, bevorzugt Komponenten aus Glas, verbunden, indem die benachbarten Flächen mit einer dünnen, optisch transparenten Kittschicht zusammen geklebt werden. Dabei wird meist ein UV-härtender optischer Kitt verwendet, welcher einen ähnlichen Brechungsindex wie die zu verbindenden Komponenten aufweist. Für eine gute optische Performance muss dabei die Grösse des Kittspalts beachtet und im optischen Design berücksichtigt werden.

Beim Bonden wird auf den optischen Kitt verzichtet und die benachbarten optischen Flächen direkt zusammengebracht. Mit diesem Verfahren lassen sich noch bessere optische Ergebnisse erreichen. Allerdings müssen die optischen Flächen sehr präzise gefertigt werden.

Beispielweise kann die Haftungskraft durch Adhäsion erzeugt werden (sogenanntes Ansprengen). Die Verbindung kann empfindlich auf Temperaturschwankungen reagieren.

Bei direkten Bonden werde hydrophile oder hydrophobe Oberflächen unter hoher Temperatur und hohem Druck zusammengebracht. Die Haftung basiert auf der Van-der-Waals-Wechselwirkung. In der Regel kann eine sehr stabile Verbindung erreicht werden.

Beim anodisches Bonden wird zusätzlich eine elektrische Spannung angelegt.

Insbesondere sind das Prisma und das Blendenelement derart verbunden, dass die Verbindung nicht zerstörungsfrei lösbar ist oder nur unter hohem Aufwand lösbar ist, wobei zum Beispiel das Verbindungsmittel zerstört wird, wenn zum Beispiel ein optischer Kitt mit Lösungsmittel entfernt wird.

Die Subbaugruppe bildet ein einteiliges Bauteil, das leicht in einem Gehäuse oder in eine Fassung montiert werden kann. Bei der Montage bleibt die relative Ausrichtung zwischen Prisma und Blendenelement erhalten. Es reicht, eines der Bauteile gegenüber der Fassung oder dem Gehäuse auszurichten. Es braucht auch nur Blendenelement an der Fassung oder dem Gehäuse befestigt zu werden.

Die Befestigung kann an den Seitenflächen des Blendenelements erfolgen. Die Subbaugruppe kann so ausgeführt sein, dass das Blendenelement quer zur Lichtdurchgangsrichtung einen grösseren Durchmesser als das Prisma aufweist.

Das Blendenelement weist eine quaderförmige Grundform auf mit einer Höhe von typischerweise etwa 0.5mm bis 1.5mm, zum Beispiel 1mm, einer Breite von typischerweise etwa 0.5mm bis 5mm, zum Beispiel 1mm und einer Länge von typischerweise 1mm-3mm, zum Beispiel etwa 3mm. Die Austrittsfläche des Primas weist dieselbe Breite wie die Breite des Blendenelements auf, aber eine geringere Länge.

Im montierten Zustand steht das Blendenelement zumindest teilweise mit dem Gehäuse oder Fassung in Kontakt, zum Beispiel mit den kleineren Seitenflächen einer quaderförmigen Grundform. Hingegen kann das Prisma frei im Raum stehen und das Gehäuse nicht berühren. Die Spiegelflächen brauchen nicht unbedingt beschichtet zu sein, da die Reflexionen aufgrund der inneren Totalreflexion an der Prisma-Luft-Grenzfläche erfolgen kann.

Das Prisma weist insbesondere eine Strahleintrittsfläche und eine Strahlaustrittsfläche auf. Die Strahlaustrittsfläche liegt bevorzugt an dem Blendenelement an. Die Strahleintrittsfläche und eine Strahlaustrittsfläche schliessen einen Winkel $\gamma$ ein. Dieser Winkel entspricht dem Winkel, um den ein einfallender Strahl von dem Prisma abgelenkt werden soll.

Die Strahleintrittsfläche und/oder die Strahlaustrittsfläche können eine Fläche von 0.5mm x 0.5mm bis 3mm x 3mm , zum Beispiel etwa 1mm x 1mm aufweisen.

Der Winkel liegt bevorzugt in einem Bereich von 10° bis 80°, weiterbevorzugt zwischen ca. 20°und 45°, zum Beispiel 22.5°.

Bevorzugt weist das Prisma zwei Reflexionsflächen auf, an denen durch die Strahleintrittsfläche eintretendes Licht reflektiert wird.

In der Regel kann einfallendes Licht durch innere Totalreflexion in dem Prisma reflektiert werden.

Mit entsprechender Wahl der Glassorte kann erreicht werden, dass die Reflexion innerhalb des Prismas durch eine interne Totalreflexion erfolgt.

Dabei muss die Bedingung $\theta > \theta_c$ für alle einfallenden Strahlen an der Grenzfläche erfüllt sein, wobei $\theta_c$ der kritische Winkel ist, bei dem der Strahl gerade nicht austritt:

$$\theta_c = \arcsin\left(\frac{n2}{n1}\right).$$

Hierbei ist n2 ist der Brechungsindex des dünneren Mediums (zum Beispiel Luft) und n1 der Brechungsindex des Prismenglases.

Der Ablenkwinkel $\gamma$ und die obige Bedingung liefern somit ein Mass für den notwendigen Brechungsindex des Prismenglases.

Falls für einen gewünschten Ablenkwinkel kein Glas zur Verfügung steht, welches diese Bedingung erfüllt, müssen die Reflexionsflächen mit einer Spiegelschicht versehen werden. Dazu wird eine dielektrische Schicht bevorzugt. Alternativ kommen auch Metallschichten oder eine Kombination aus Metallschicht und dielektrischer Schicht in Frage.

Mit einer Beschichtung ist eine Reflexion auch bei Winkeln möglich, bei denen keine innere Totalreflexion mehr erfolgt.

In einer vorteilhaften Ausführung der Subbaugruppe sind das Prisma und/oder das Blendenelement aus Glas gefertigt.

Mit glasgefertigten Bauteilen lassen sich sehr ebene Flächen, insbesondere Spiegelflächen, realisieren und somit wird eine hohe Abbildungsgüte ermöglicht.

Insbesondere weist das Prisma polierte Glasflächen auf.

Eine Verspiegelung der Reflexionsflächen ist nicht unbedingt notwendig.

Die Aufgabe wird ausserdem gelöst durch ein Objektiv, insbesondere für ein langes und schlankes optisches Bildübertragungssystem, zum Beispiel ein Endoskop. Das Objektiv umfasst mindestens eine Subbaugruppe wie sie oben beschrieben wurde.

Die Subbaugruppe ist insbesondere derart in ein zylindrisches Gehäuse montiert, dass die Austrittsfläche des Prismas senkrecht zur Längsachse des Gehäuses angeordnet ist.

In einer bevorzugten Ausführung des Objektivs umfasst das Objektiv eine, bevorzugt plankonkave, Eintrittslinse und/oder mindestens eine Abbildungslinse.

Die Subbaugruppe und die Linsen sind in einem gemeinsamen, insbesondere zylindrischen, Gehäuse angeordnet.

Die optische Achse der Eintrittslinse ist insbesondere senkrecht zur Eintrittsfläche des Prismas angeordnet.

Das Objektiv besitzt also einen schrägen Blickwinkel, da die optische Achse der Eintrittslinse gegenüber der Längsachse des Gehäuses geneigt ist.

Die optische Achse der Abbildungslinse ist insbesondere senkrecht zur Austrittsfläche des Prismas angeordnet. Das Objektiv weist bevorzugt drei Abbildungslinsen auf, deren optische Achsen miteinander fluchten.

In dem Objektiv kann zwischen Eintrittslinse und Subbaugruppe ein Abstimmring angeordnet sein. Mit dem Abstimmring können Abbildungsfehler kompensiert werden, die zum Beispiel dadurch auftreten können, dass die Reflexionsflächen des Prismas einen unpassenden Abstand aufweisen. Ein Lichtstrahl, der die Eintrittslinse zentral durchläuft, trifft dann nicht auf das Zentrum der Abbildungslinse. Ein Zentrierfehler kann zu Abbildungsfehlern führen.

Über die Variation des Abstandes zwischen Eintrittslinse und Subbaugruppe kann eine Zentrierung erreicht werden, welche für eine geforderte Abbildungsqualität nötig ist. Mit dem Abstimmring kann der für die optimale Zentrierung passende Abstand festgelegt werden. Eine präzise Zentrierung kann auch über eine Verschiebung der Eintrittslinse erreicht werden, auch ohne Abstimmring.

Die Aufgabe wird ausserdem gelöst durch ein Bildübertragungssystem, zum Beispiel ein Endoskop, mit mindestens einem Objektiv wie es oben beschrieben wurde. Das Objektiv kann insbesondere lösbar an einem Systemgehäuse befestigt sein. Das Objektiv kann ausgetauscht, gewartet und/oder gereinigt werden.

Ein Bildübertragungssystem kann auch eine Systemgehäuse aufweise, an welchem zwei Objektive angebracht sind, sodass zwei Bildübertragungskanäle um Einsatz kommen. Mit zwei Bildübertragungskanälen können beispielsweise dreidimensionale Darstellungen realisiert werden.

Das Bildübertragungssystem kann aus einem Baukasten zusammengestellt werden, zu dem mehrere Objektive gehören können, zum Beispiel mit unterschiedlicher Blickwinkelneigung, die jeweils wahlweise mit dem Systemgehäuse verbunden werden können.

Bei einem Verfahren zum Fertigen einer Subbaugruppe wie oben beschrieben, wird ein Prisma mit einer Strahleintrittsfläche und einer Strahlaustrittsfläche derart an einem Blendenelement fixiert, dass die Strahlaustrittsfläche an dem Blendenelement anliegt und die Blendenöffnung abdeckt. Die Fixierung erfolgt derart, dass das Prisma mit dem Blendenelement fest verbunden ist.

Insbesondere wird eine Verbindung hergestellt, die nicht zerstörungsfrei lösbar ist oder nur unter hohem Aufwand lösbar ist, wobei zum Beispiel das Verbindungsmittel zerstört wird, wenn zum Beispiel ein optischer Kitt mit Lösungsmittel entfernt wird.

Vorteilhafterweise wird das Prisma an das Blendenelement gekittet. Als optisches Kittmittel kann beispielsweise NOA61 (Norland Optical Adhesive 61) verwendet werden.

Es kann auch eine Verbindung über hydrophiles Bonden hergestellt werde. Bei dem hydrophilen Bonden ohne Kitt handelt es sich um eine mineralische Fügetechnik, insbesondere für Gläser und transparente kristalline Materialien. Diese Verbindungstechnik garantiert volle Transparenz an der Verbindungstelle, hohe mechanische und thermische Stabilität und erlaubt eine präzise Ausrichtung der Grenzflächen.

In einer vorteilhaften Ausführung des Verfahrens wird ein Blendenwafer bereitgestellt. Dieser weist bevorzugt ein mit Schwarzchrom beschichtetes Blendensubstrats auf. Das Substrat kann aus Glas, zum Beispiel D263T Dünnglas oder Quarzglas, bestehen.

Der Blendenwafer besitzt mindestens eine Blendenöffnung, bevorzugt jedoch eine Anordnung von einer Vielzahl von Blendenöffnungen.

Ausserhalb der Blendenöffnungen ist der Blendenwafer licht undurchlässig. Das Blendensubstrat, zum Beispiel aus Glas, kann dafür mit Schwarzchrom beschichtet sein.

Zudem wird mindestens ein Prismengrundkörper mit einer Strahleintrittsfläche und eine Strahlaustrittsfläche bereitgestellt.

Der Blendenwafer kann eine Dicke zwischen0.5mm und 1.5mm, bevorzugt von etwa 0.7mm aufweisen.

Die Fläche des Blendenwafers kann zwischen 10mm x 10mm und 100mm x 100mm liegen, bevorzugt bei etwa 45mm x 45mm.

Der Blendendurchmesser kann zwischen 0.1mm und 1mm liegen, bevorzugt bei etwa 0.4mm. Die Beschichtung weist bevorzugt eine optische Dichte von grösser als 3 auf.

Typischerweise wird für jede Blendenöffnung oder für eine Reihe von Blendenöffnungen des Blendenwafers je ein Prismengrundkörper bereitgestellt. Der Prismengrundkörper ist bevorzugt stabförmig und weist insbesondere eine Länge von 10mm-100mm, zum Beispiel 40mm auf.

Es erfolgt ein Verbinden von Blendenwafer und Prismengrundkörper, wobei je ein Prismengrundkörper auf eine Blendenöffnung oder auf eine Reihe von Blendenöffnungen gesetzt wird.

Die Kittspalt-Dicke kann kontrolliert werden mit Microbeads, Dünnfäden, Dünnfolien, Beschichten, zum Beispiel Bedampfen, oder mit einem Abstandhalte-Werkzeug.

Die Kittspaltdicke beträgt zwischen 0.01mm und 0.05mm, zum Beispiel 0.03mm.

Danach wird der Blendenwafer geschnitten, zum Beispiel mit einer Wafersäge, sodass eine oder mehrere einzelne Subbaugruppen erhalten werden. Gegebenenfalls wird beim Schneiden auch ein Prismengrundkörper durchtrennt, der eine Reihe von Blendenöffnungen abdeckt.

Der Blendenwafer kann mit Hilfslinien ausgestattet sein, die ein Positionieren des Prismengrundkörpers erleichtern und die Trennlinien zum Schneiden des Blendenwafer vorgeben. Alternativ und/oder zusätzlich können Markierungen vorgesehen sein, die das Positionieren zum Beispiel einer Schneidemaschine erleichtern. Hilfslinien und/oder Markierungen können in die Beschichtung eingeritzt sein oder lithographisch hergestellt sein..

In einem Verfahren zum Montieren eines Objektivs, wie es weiter oben beschrieben ist, wird eine Subbaugruppe bereitgestellt, bevorzugt in einem Verfahren wie es oben beschrieben ist, und die Subbaugruppe in ein Gehäuse fixiert, insbesondere eingeklebt, wird.

Bei der Montage des Objektivs kann zunächst eine Eintrittslinse am Gehäuse befestigt werden und bevorzugt ein Abstimmring zwischen Eintrittslinse und Subbaugruppe positioniert werden. Der Abstimmring legt den Abstand zwischen Eintrittslinse und Subbaugruppe fest, sodass nach Möglichkeit kein Zentrierfehler auftritt.

Es kann auch zunächst die Subbaugruppe am Gehäuse befestigt werden, dann eine Eintrittslinse gegenüber der Subbaugruppe verschoben werden, bis eine optimale Position gefunden ist. Anschliessend wird die Eintrittslinse am Gehäuse befestigt.

Während des Verschiebens kann kontrolliert werden, ob ein Zentrierfehler vorliegt. Es kann die beste Position der Eintrittslinse für eine optimale Abbildungsqualität eingestellt werden.

Vorteilhafterwiese wird für die Verklebung von der Subbaugruppe in einem Gehäuse Klebstoff durch eine Öffnung im Gehäuse in den Raum zwischen Subbaugruppe und Gehäuse eingebracht und die Öffnung durch den Klebstoff wieder verschlossen.

In einer vorteilhaften Ausführung des Verfahrens wird die Subbaugruppe mittels eines Einschiebewerkzeugs in das Gehäuse geschoben. Das Einschiebewerkzeug weist einen Montagekopf mit einer Montageausnehmung zur Aufnahme des Blendenelements auf.

Die Montageausnehmung kann die Form einer Nut aufweisen, in welcher ein Blendenelement mit quaderförmiger Grundform Platz findet.

Das Einschiebewerkzeug weist ausserdem einen Führungskörper auf, dessen äusserer Durchmesser dem inneren Durchmesser des Gehäuses entspricht, sodass das Einschiebewerkzeug ohne Verkippung in das Gehäuse eingeführt werden kann. Die Ausrichtung der Subbaugruppe in dem Gehäuse ist daher durch den Montagekopf, bzw. der Richtung der Montageausnehmung, vorgegeben und es kann eine sehr präzise Positionierung erreicht werden.

Das Einschiebewerkzeug kann einen Führungsstift besitzen, der radial von dem Führungskörper des Einschiebewerkzeugs wegweist und die Ausrichtung der Subbaugruppe, insbesondere des Prismas, anzeigt. Bevorzugt greift der Führungsstift in einen Schlitz ein, der im Gehäuse vorgesehen ist, wenn die Subbaugruppe bezüglich des Gehäuses und der Neigung der Eintrittslinse die richtige Ausrichtung aufweist.

Ein Einschiebewerkzeug zum Einschieben einer Subbaugruppe in ein Gehäuse in einem Verfahren zum Montieren eines Objektives wie oben beschrieben, weist einen Montagekopf mit einer Montageausnehmung zur Aufnahme der Subbaugruppe auf und einen Führungskörper, dessen äusserer Durchmesser dem inneren Durchmesser des Gehäuses entspricht. Bevorzugt weist das Einschiebewerkzeug einen Führungsstift, der die Ausrichtung der Subbaugruppe anzeigt und mit einem Schlitz in Gehäuse zusammenwirken kann.

Bevorzugte Ausführungsbeispiele der Erfindung sind in der nachfolgenden Beschreibung anhand der beigefügten Zeichnungen näher erläutert. Dabei sind entsprechende Elemente mit übereinstimmenden Bezugszeichen versehen.

Es zeigen

| | |
|---|---|
| Figur 1 | eine Subbaugruppe in seitlicher Schnittansicht; |
| Figur 2 | ein Objektiv in perspektivischer Schnittansicht; |
| Figur 3 | eine schematische Darstellung eines Strahlverlaufs in einem Objektiv in seitlicher Schnittansicht; |
| Figur 4 | eine schematische Darstellung von Objektiven im Vergleich in seitlicher Schnittansicht; |
| Figuren 5a, 5b | Subbaugruppen in perspektivischer Ansicht; |
| Figuren 6a-6b | schematische Darstellungen von Fertigungsschritten; |
| Figur 7 | ein Objektiv und ein Einschiebewerkzeug in Explositionsdarstellung. |

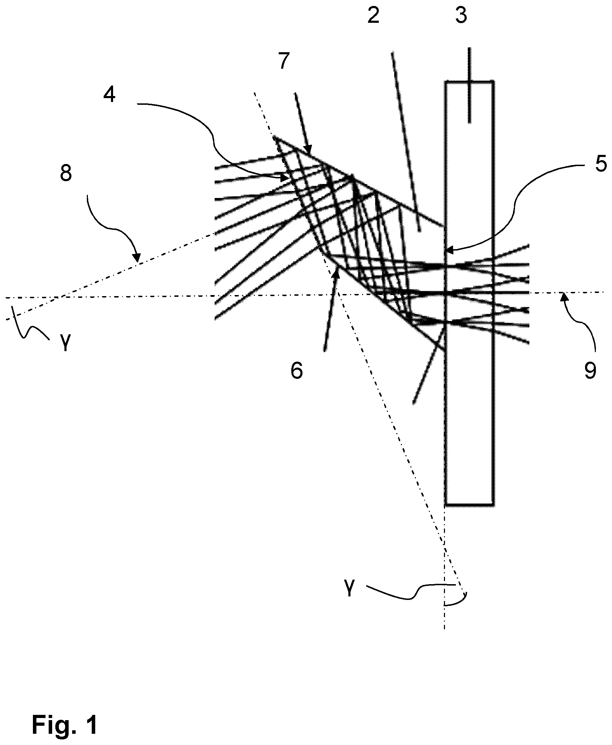

Figur 1 zeigt eine Subbaugruppe 1 in seitlicher Schnittansicht. Die Subbaugruppe 1 umfasst ein Prisma 2 und ein Blendenelement 3, die fest miteinander verbunden sind, bevorzugt über eine Verkittung oder über ein direktes Bonding.

Das Prisma 2 besitzt eine Strahleintrittsfläche 4 und eine Strahlaustrittsfläche 5. Die Strahlaustrittsfläche 5 liegt an dem Blendenelement 3 an.

Die Strahleintrittsfläche 4 und die Strahlaustrittsfläche 5 schliessen einen Winkel $\gamma$ ein, der dem Winkel $\gamma$ zwischen der Richtung 8 eines einfallenden Strahls und der Richtung 9 eines ausfallenden Strahls entspricht.

Das Prisma 2 weist zwei Reflexionsflächen 6, 7 auf. Diese können spiegelbeschichtet sein.

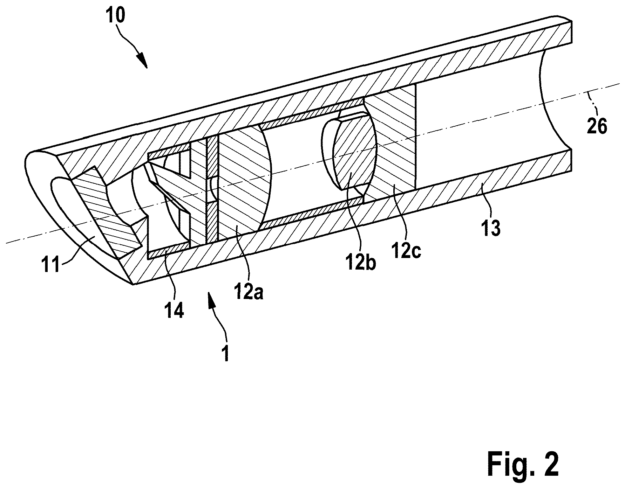

Figur 2 zeigt ein Objektiv 10 in perspektivischer Schnittansicht. Das Objektiv 10 umfasst eine Subbaugruppe 1, eine plankonkave Eintrittslinse 11 und drei Abbildungslinsen 12a, 12b, 12c, die in einem Gehäuse 13 angeordnet sind.

Die konkave Fläche der Eintrittslinse 11 steht nicht senkrecht auf der Längsachse 26 des Gehäuses 13, sodass das Objektiv 10 abgewinkelt ist.

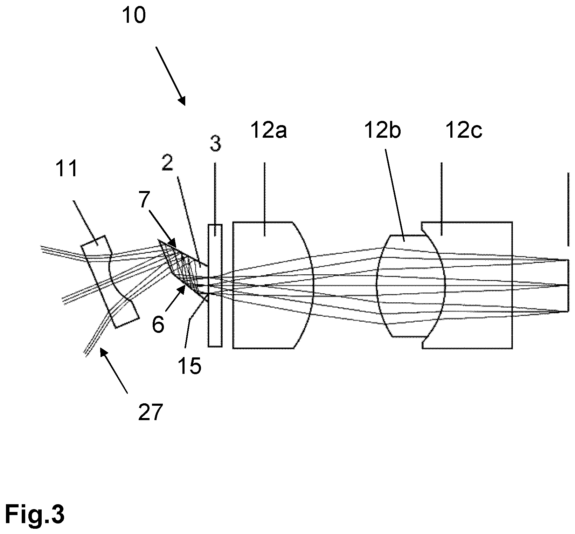

Figur 3 zeigt eine schematische Darstellung eines Strahlverlaufs in einem Objektiv 10 in seitlicher Schnittansicht.

Die Strahlenbündel 27 verlaufen, beispielweise mit einem Feldwinkel von ±35°, durch die plankonkave Eintrittslinse 11. Danach wird der Strahl durch das Prisma 2 geleitet, wobei eine Totalreflexion an den Reflexionsflächen 6, 7 stattfindet, wodurch der Strahl umgelenkt wird. Der Strahl durchläuft dann die Blendenöffnung 15 des Blendenelements 3. Anschliessend durchläuft der Strahl drei Abbildungslinsen 12a, 12b und 12c.

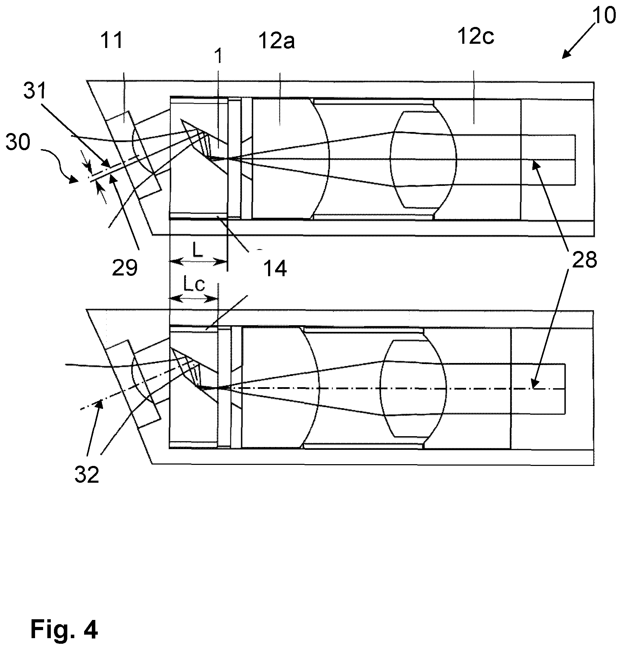

Figur 4 zeigt eine schematische Darstellung von Objektiven 10 im Vergleich in seitlicher Schnittansicht.

In dem Objektiv 10 in der oberen Darstellung sind die Eintrittslinse 11 und die Subbaugruppe 1 so zueinander angeordnet, dass ein zentral abgebildeter Lichtstrahl 28 einem einlaufenden Lichtstrahl 29 entspricht, der einen Versatz 30 zur zentralen Achse 31 der Eintrittslinse 11 aufweist.

In dem Objektiv 10 der oberen Darstellung ist eine Abstimmring 14 der Länge L angeordnet.

Wird dieser durch einen Abstimmring 14 der Länge Lc ersetzt, wie in der unteren Darstellung gezeigt, ist der Abstand zwischen Eintrittslinse 11 und Subbaugruppe 1 derart verändert, dass eine zentral einlaufender Strahl 32 als zentral auslaufender Strahl 28 abgebildet wird.

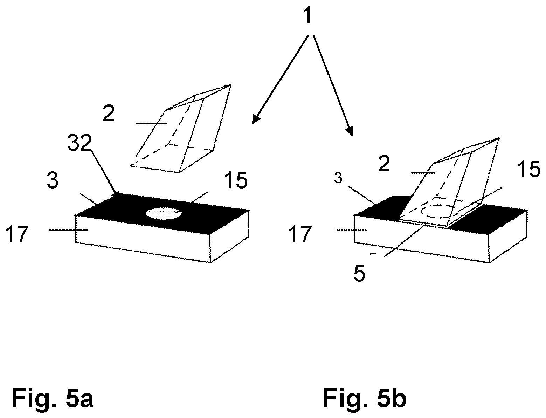

Figuren 5a, 5b zeigen Subbaugruppen 1 in perspektivischer Ansicht. Die Subbaugruppe besteht aus einem Prisma 2 und einem Blendenelement 3.

Das Blendenelement 3 weist eine Blendenöffnung 15 auf.

Auf einem Blendensubstrat 17 ist eine lichtundurchlässige Beschichtung 32 aufgebracht.

Nach der Montage, liegt die Strahlaustrittsfläche 5 des Prismas 2 fest an dem Blendenelement 3 an und deckt die Blendeöffnung 15 ab.

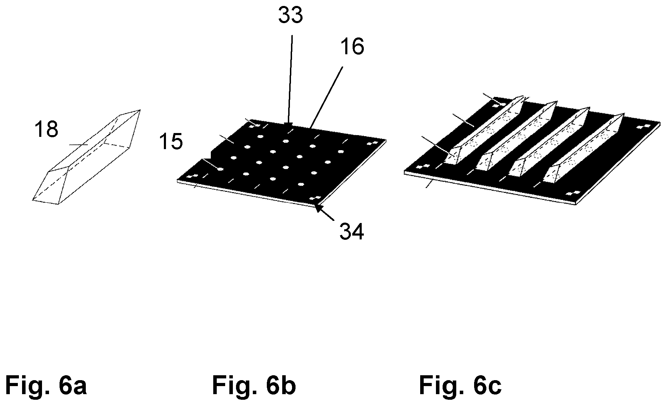

Figuren 6a-6b zeigen schematische Darstellungen von Fertigungsschritten. Zunächst wird mindestens ein Prismengrundkörper 18 zur Verfügung gestellt gemäss Figur 6a.

Ausserdem wird ein Blendenwafer 16 mit einer Vielzahl von Blendenöffnungen 15 bereitgestellt.

Auf dem Blendenwafer 16 befinden sich Hilfslinien 33 und Markierungen 34.

Die Hilfslinien helfen dabei, die Prismengrundkörper 18 so auf dem Blendenwafer 16 anzuordnen, dass je eine Reihe von Blendenöffnungen 15 gleichmässig von einem Prismengrundkörper 18 abgedeckt wird.

Die Prismengrundkörper 18 werden fest mit dem Blendenwafer 16 verbunden.

Anschliessend kann der Blendenwafer 16 parallel zu den stabförmigen Prismengrundkörpern 18 geschnitten werden, wobei die Markierungen 34 als Orientierung dienen.

Abschliessend werden die Prismengrundkörper zusammen mit dem Blendenwafer 16 geschnitten, um einzelne Subbaugruppen 1, wie sie in Figur 5b dargestellt ist, zu erhalten.

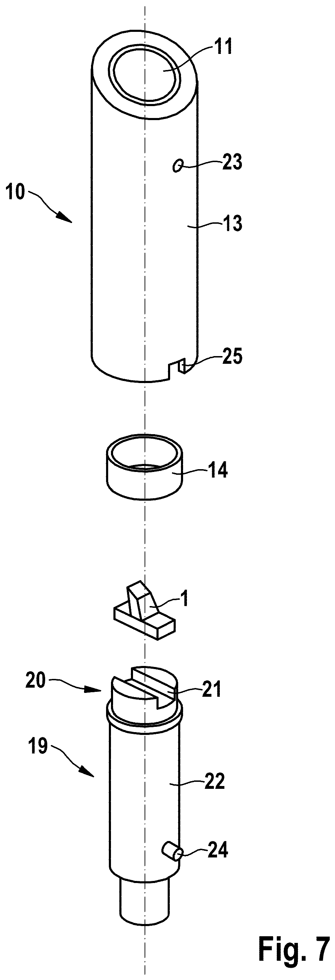

Figur 7 zeigt ein Objektiv 10 und ein Einschiebewerkzeug 19 in Explosionsdarstellung.

Das Einschiebewerkzeug 19 weist einen Montagekopf 20 mit einer Montageausnehmung 21 zur Aufnahme des Blendenelement 3 auf und einen Führungskörper 22, dessen äusserer Durchmesser dem inneren Durchmesser des Gehäuses 13 entspricht, so dass das Einschiebewerkzeug 19 mit der Subbaugruppe passgenau in das Gehäuse 13 geschoben werden kann.

Das Einschiebewerkzeug 19 besitzt einen Führungsstift 24, der radial von dem Führungskörper 22 des Einschiebewerkzeugs 19 wegweist. Der Führungsstift 24 kann in einen Schlitz 25, der im Gehäuse 13 vorgesehen ist, eingreifen, wenn die Subbaugruppe 1 bezüglich des Gehäuses 13 die richtige Ausrichtung aufweist.

Am Gehäuse 13 ist eine Eintrittslinse 11 befestigt. Ein Abstimmring 14 wird zwischen Eintrittslinse 11 und Subbaugruppe 1 positioniert.

**Patentansprüche**

1. Subbaugruppe (1) für ein Objektiv (10), insbesondere für ein langes und schlankes optisches Bildübertragungssystem zum Beispiel ein Endoskop (100), umfassend ein Prisma (2) und ein Blendenelement (3),

   wobei das Prisma (2) und das Blendenelement (3) fest miteinander verbunden sind, bevorzugt mittels einer Verkittung oder über ein direktes Bonding, wobei das Blendenelement (3) quer zur Lichtdurchgangsrichtung einen grösseren Durchmesser als das Prisma (2) aufweist, wobei das Prisma (2) eine Strahleintrittsfläche (4) aufweist und eine Strahlaustrittsfläche (5) und die Strahlaustrittsfläche (5) an dem Blendenelement (3) anliegt, wobei das Blendenelement (3) eine quaderförmige Grundform aufweist mit einer Höhe, einer Breite und einer Länge,
   **dadurch gekennzeichnet, dass**
   die Austrittsfläche des Primas (2) dieselbe Breite wie die Breite des Blendenelements aufweist, aber eine geringere Länge, wobei sich die Breite und die Länge der Strahlaustrittsfläche in die gleichen Richtungen wie die Breite und die Länge der Grundform erstrecken.

2. Subbaugruppe (1) gemäss Anspruch 1, wobei das Prisma (2) eine Strahleintrittsfläche (4) aufweist und eine Strahlaustrittsfläche (5) und die Strahlaustrittsfläche (5) an dem Blendenelement (3) anliegt, und wobei die Strahleintrittsfläche (4) und die Strahlaustrittsfläche (5) einen Winkel $\gamma$ zwischen 10° und 80° einschliessen.

3. Subbaugruppe (1) gemäss Anspruch 1, wobei das Prisma (2) zwei Reflexionsflächen (6, 7) aufweist, die nicht beschichtet sind.

4. Subbaugruppe (1) gemäss Anspruch 1, wobei das Prisma (2) zwei Reflexionsflächen (6, 7) aufweist, die spiegelbeschichtet sind.

5. Subbaugruppe (1) gemäss einem der vorhergehenden Ansprüche, wobei das Prisma (2) und/oder das Blendenelement (3) aus Glas gefertigt sind.

6. Objektiv (10), insbesondere für ein langes und schlankes optisches Bildübertragungssystem, zum Beispiel ein Endoskop (100), umfassend mindestens eine Subbaugruppe (1) gemäss einem der vorhergehenden Ansprüche.

7. Objektiv (10) gemäss Anspruch 6, wobei das Objektiv (10) ein Gehäuse (13), eine, bevorzugt plankonkave, Eintrittslinse (11) und/oder mindestens eine Abbildungslinse (12a, 12b, 12c) umfasst, wobei die Eintrittslinse (11) die und/oder die mindestens eine Abbildungslinsen in dem Gehäuse (13) angeordnet sind.

8. Objektiv (10) gemäss Anspruch 6 oder 7, wobei das Objektiv (10) einen Abstimmring (14) umfasst und der Abstimmring (14) zwischen Eintrittslinse (11) und Subbaugruppe (1) angeordnet ist.

9. Bildübertragungssystem (100), zum Beispiel Endoskop, mit mindestens einem Objektiv (10) gemäss einem der Ansprüche 7-8.

**Claims**

1. Subassembly (1) for an objective (10), in particular for a long and slim optical image transmission system, for example an endoscope (100), comprising a prism (2) and an aperture element (3),

   wherein the prism (2) and the aperture element (3) are firmly connected to each other, preferably by means of a putty or by direct bonding, wherein the aperture element (3) has a larger diameter than the prism (2) transverse to the direction of light transmission, wherein the prism (2) has a beam entry surface (4) and a beam exit surface (5) and the beam exit surface (5) rests against the aperture element (3), wherein the aperture element (3) has a cuboid basic shape with a height, a width and a length, **characterized in that** the exit surface of the prism (2) has the same width as the width of the aperture element, but a shorter length, wherein the width and length of the beam exit surface extend in the same directions as the width and length of the basic shape.

2. Subassembly (1) according to claim 1, wherein the prism (2) has a beam entry surface (4) and a beam exit surface (5) and the beam exit surface (5) rests against the aperture element (3), and wherein the beam entry surface (4) and the beam exit surface (5) form an angle γ between 10° and 80°.

3. Subassembly (1) according to claim 1, wherein the prism (2) has two reflection surfaces (6, 7) which are uncoated.

4. Subassembly (1) according to claim 1, wherein the prism (2) has two reflective surfaces (6, 7) which are mirror-coated.

5. Subassembly (1) according to one of the preceding claims, wherein the prism (2) and/or the aperture element (3) are made of glass.

6. Objective (10), in particular for a long and slender optical image transmission system, for example an endoscope (100), comprising at least one subassembly (1) according to one of the preceding claims.

7. Objective (10) according to claim 6, wherein the objective (10) comprises a housing (13), a preferably plano-concave entrance objective (11) and/or at least one imaging lens (12a, 12b, 12c), wherein the entrance lens (11) and/or the at least one imaging lens are arranged in the housing (13).

8. Objective (10) according to claim 6 or 7, wherein the objective (10) comprises a tuning ring (14) and the tuning ring (14) is arranged between the entrance lens (11) and the subassembly (1).

9. Image transmission system (100), for example an endoscope, with at least one objective (10) according to one of claims 7-8.

**Revendications**

1. Sous-ensemble (1) pour un objectif (10), en particulier pour un système optique de transmission d'images long et mince, par exemple un endoscope (100), comprenant un prisme (2) et un élément d'ouverture (3),

   le prisme (2) et l'élément d'ouverture (3) étant reliés de manière fixe l'un à l'autre, de préférence par collage ou par liaison directe, l', l'élément d'ouverture (3) présentant, transversalement à la direction de passage de la lumière, un diamètre supérieur à celui du prisme (2) ,

le prisme (2) présentant une surface d'entrée du faisceau (4) et une surface de sortie du faisceau (5) et la surface de sortie du faisceau (5) étant adjacente à l'élément d'obturation (3), l'élément d'obturation (3) présentant une forme de base parallélépipédique avec une hauteur, une largeur et une longueur, **caractérisé en ce que** la surface de sortie du prisme (2) présente la même largeur que la largeur de l'élément d'obturation, mais une longueur inférieure, la largeur et la longueur de la surface de sortie du faisceau s'étendant dans les mêmes directions que la largeur et la longueur de la forme de base.

2. Sous-ensemble (1) selon la revendication 1, le prisme (2) présentant une surface d'entrée du faisceau (4) et une surface de sortie du faisceau (5) et la surface de sortie des rayons (5) est adjacente à l'élément d'ouverture (3), et dans lequel la surface d'entrée des rayons (4) et la surface de sortie des rayons (5) forment un angle $\gamma$ compris entre 10° et 80°.

3. Sous-ensemble (1) selon la revendication 1, le prisme (2) présentant deux surfaces de réflexion (6, 7) qui ne sont pas revêtues.

4. Sous-ensemble (1) selon la revendication 1, le prisme (2) présentant deux surfaces réfléchissantes (6, 7) qui sont revêtues d'un miroir.

5. Sous-ensemble (1) selon l'une des revendications précédentes, dans lequel le prisme (2) et/ou l'élément d'obturation (3) sont en verre.

6. Objectif (10), en particulier pour un système optique de transmission d'images long et mince, par exemple un endoscope (100), comprenant au moins un sous-ensemble (1) selon l'une des revendications précédentes.

7. Objectif (10) selon la revendication 6, l'objectif (10) comprenant un boîtier (13), une lentille d'entrée (11), de préférence plane-concave, et/ou au moins une lentille de formation d'image (12a, 12b, 12c), la lentille d'entrée (11) et/ou la ou les lentilles de formation d'image étant disposées dans le boîtier (13).

8. Objectif (10) selon la revendication 6 ou 7, l'objectif (10) comprenant une bague de réglage (14) et la bague de réglage (14) étant disposée entre la lentille d'entrée (11) et le sous-ensemble (1).

9. Système de transmission d'images (100), par exemple endoscope, avec au moins un objectif (10) selon l'une des revendications 7 à 8.

**Fig. 1**

**Fig. 2**

**Fig.3**

**Fig. 4**

**Fig. 5a**             **Fig. 5b**

Fig. 6a          Fig. 6b          Fig. 6c

11

10

23

13

25

14

1

20

21

19

22

24

# Fig. 7

**EP 4 018 229 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4138192 A
- US 4850342 A
- US 5519532 A
- CN 105403992 A
- DE 3640186 A1